# EUROPEAN PATENT APPLICATION

(11) **EP 2 422 695 A2**
(43) Date of publication of application: **29.02.2012**
(21) Application number: 11178203.3
(22) Date of filing: 19.08.2011
(51) Int. Cl.: A61B 5/00

(54) **Monitoring patients receiving care**

(30) Priority: 24.08.2010 US 862179
(71) Applicant: General Electric Company, Schenectady, NY 12345 (US)
(72) Inventor: Rantala, Borje, 00670 Helsinki (FI)
(74) Representative: Illingworth-Law, William Illingworth

(57) **Abstract**

A method, device, and computer program product for monitoring a subject (110) receiving care are disclosed. To enable creation of different clinical applications (115) with visually informative user interfaces similar to each other, a monitoring apparatus (100) is provided with a generic monitor module (114) serving as a template for creation of monitoring instances. At least one clinical application (115) may be created from the generic monitor module (114), wherein each clinical application defines a care process for caring a subject, and a subject-specific monitoring instance may be produced from any of the at least one clinical application (115), thereby to obtain a runtime monitoring instance. The runtime monitoring instance may then be employed for monitoring the subject (110) online. (FIG. 1)

## Description

### Background of the Invention

This disclosure relates generally to patient monitoring. More particularly, the present invention relates to monitoring a subject that receives care according to a certain care process planned for him/her.

Patient monitors are electronic devices designed to display physiological information about a subject. Electrocardiogram (ECG), electroencephalogram (EEG), plethysmographic signals, and signals related to blood pressure, temperature, and respiration represent typical physiological information contained in full-size patient monitors. Patient monitors are typically also furnished with alarming functionality to alert the nursing staff when a vital sign or physiological parameter of a patient exceeds or drops below a preset limit. Alarms are normally both audible and visual effects aiming to alert the staff to a life-threatening condition or to another event considered vital. In most monitors, the alarm limits may be defined by the user, since the limits typically depend on patient etiology, age, gender, medication, and various other subjective factors. Each specific physiological parameter, such as heart rate or blood pressure, may also be assigned more than one alarm limit.

In addition to individual sensor/parameter alarms, patient monitors can be configured to raise combinatory alarms. That is, several physiological parameters may be used to determine a combined index and to give an alarm when the combined index fulfills a specific criterion. The combinatory alarms may range from simple combinations like "low heart rate and low arterial pressure" to complex rule-based scenarios used in various clinical support systems designed to assist the medical staff in the course of a care process. The clinical support systems help the medical staff use standardized guidelines and treatment procedures and support the medical staff in clinical decision-making.

The clinical support systems are normally designed for one or more specific diseases or medical conditions, such as sepsis, since it is important to ensure that care is given according to the existing recommendations for the particular disease in question. Although this provides substantial support for the medical staff in view of the diseases or medical conditions concerned, it also makes the care processes inflexible since the built-in intelligence of one monitor/system is for the specific medical condition only and cannot be adapted to other medical conditions. Furthermore, as each monitor/system is designed for a specific medical condition, the use of multiple monitors is normally challenging, due to the different human-machine interfaces. Due to the complexity of the built-in intelligence, it may also be difficult for a clinician to grasp the connection between the behavior of the clinical index displayed by the system and the underlying physiological behavior of the patient.

### Brief Description of the Invention

The above-mentioned problems are addressed herein which will be comprehended from the following specification. The monitoring mechanism disclosed is based on a discovery that at a higher level of abstraction, many care processes obey similar logic and can thus be modified from the same generic monitor template, if certain prerequisites are taken into account in the template, such as the need to track the ability of the subject to receive care according to a certain care phase. This generic monitor template then allows creation of a set of different clinical applications having visually informative user interfaces that are similar to each other regardless of the different applications.

In an embodiment, a method for monitoring a subject receiving care comprises providing a monitoring apparatus with a generic monitor module serving as a template for creation of monitoring instances and creating at least one clinical application from the generic monitor module, wherein each clinical application defines a care process for caring a subject. The method further comprises producing a subject-specific monitoring instance from a clinical application, thereby to obtain a runtime monitoring instance, wherein the clinical application is any of the at least one clinical application and employing the runtime monitoring instance for monitoring the subject online.

In another embodiment, an apparatus for monitoring a subject receiving care comprises a generic monitor module serving as a template for creation of monitoring instances, a first configuration module adapted to allow a user to create at least one clinical application from the generic monitor module, wherein each clinical application defines a care process for caring a subject, a second configuration module adapted to allow a user to create a subject-specific monitoring instance from a clinical application, thereby to obtain a runtime monitoring instance, wherein the clinical application is any of the at least one clinical application, wherein the apparatus is configured to execute the runtime monitoring instance for monitoring the subject online.

In a still further embodiment, a computer program product for monitoring a subject receiving care comprises a generic monitor module serving as a template for creation of monitoring instances and a first program product portion adapted to allow a user to create at least one clinical application from the generic monitor module, wherein each clinical application defines a care process for caring a subject. The program product further includes a second program product portion adapted to allow a user to create a subject-specific monitoring instance from the clinical application, thereby to obtain a runtime monitoring instance, wherein the clinical application is any of the at least one clinical application and the runtime monitoring instance is executable by a monitoring apparatus for monitoring the subject online.

Various other features, objects, and advantages of the invention will be made apparent to those skilled in the art from the following detailed description and accompanying drawings.

### Brief Description of the Drawings

FIG. 1 illustrates one embodiment of an apparatus or system for monitoring a subject;

FIG. 2 is a flow diagram illustrating one embodiment of the sequence of steps to be carried out for monitoring a subject in phased care;

FIG. 3 illustrates one embodiment of the structure and operation of the generic monitor module stored in a monitoring apparatus;

FIG. 4 is a flow diagram illustrating an example of the general operation of a clinical application created from the generic monitor module;

FIG. 5 illustrates an example of a generic layout of a screen page for illustrating the clinical condition of the subject in terms of the planned care;

FIGs. 6 and 7 illustrate examples of the applications of the screen page of FIG. 5; and

FIG. 8 illustrates the units of the control and processing unit in logical sense and in view of the creation of a subject-specific clinical application for monitoring the subject.

### Detailed Description of the Invention

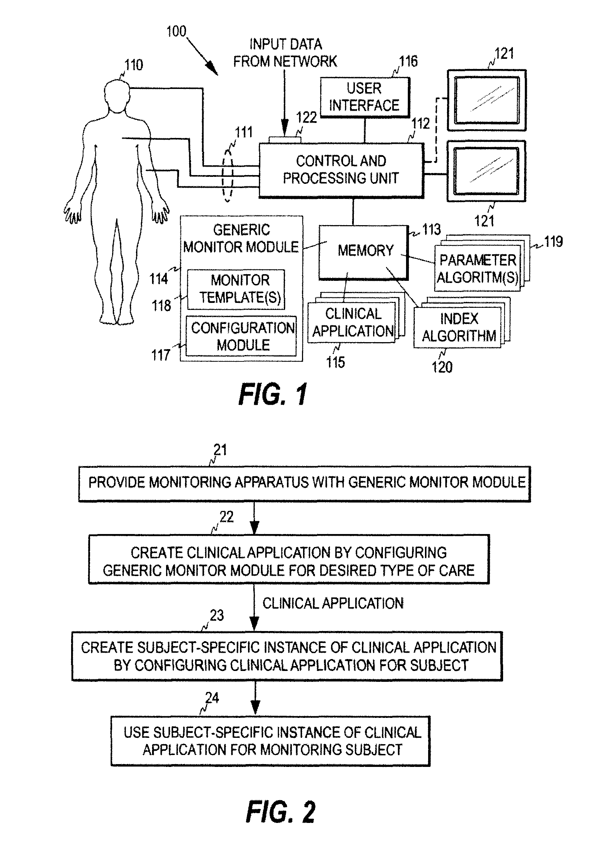

FIG. 1 illustrates one embodiment of a monitoring apparatus/system 100 for monitoring a subject 110. A monitoring apparatus/system normally acquires a plurality of physiological signals 111 from the subject, where one physiological signal corresponds to one measurement channel. The physiological signals typically comprise several types of signals, such as ECG, EEG, blood pressure, respiration, and plethysmographic signals. Based on the raw real-time physiological signal data obtained from the subject, a plurality of physiological parameters may be determined. A physiological parameter here refers to a variable calculated from the waveform data of one or more of the physiological signals acquired from the subject. If a physiological parameter is derived from more than one physiological signal, i.e. from more than one measurement channel, the said physiological signals are usually of the same signal type. The physiological parameter may thus also represent a waveform signal value determined over a predefined period of time, although the physiological parameter is typically a distinct parameter derived from one or more measurement channels, such as heart rate derived from an ECG signal or an Sp02 value derived from a plethysmographic signal. Each signal parameter may be assigned one or more alarm limits to alert the nursing staff when the parameter reaches or crosses the alarm limit.

The physiological signals 111 acquired from the subject 110 are supplied to a control and processing unit 112 through a pre-processing stage (not shown) comprising typically an input amplifier and a filter, for example. The control and processing unit converts the signals into digitized format for each measurement channel. The digitized signal data may then be stored in the memory 113 of the control and processing unit.

It is assumed here that the subject is receiving care in different phases. That is, the care planned for the subject comprises a well-defined sequence of care phases. To enable monitoring of subjects in different types of care, the apparatus is provided with a generic monitor module 114, which is a software system from which clinical application instances 115 may be created. The clinical application instances, which are used in the runtime environment to monitor the subject, may be subject-specific.

The generic monitor module is a combination of a state machine and a rule based engine: in each clinical application an instance created from the module handles defined transitions between the phases of the care (state machine) and executes specified monitoring rules during each phase (rule based engine). The generic monitor module is configurable through a user interface 116 of the apparatus in the sense that the user may create monitor instances for runtime monitoring environment. In logic sense, the generic monitor module thus comprises a configuration unit 117 by which the user may create the runtime instances from monitor templates 118 through user interface 116. The configuration unit may also be regarded as an instance construction unit.

Each clinical application instance may utilize one or more parameter algorithms 119 adapted to record, when executed by the control and processing unit, the time series of the physiological parameters needed in various phases of the planned care. The obtained time series of the physiological parameters may be stored in the memory. Although the apparatus typically records a plurality of parameters, part of the parameter sequences may be received from devices external to the monitoring apparatus.

For the determination of the ability of the subject to receive care according to a certain care phase, the control and processing unit may further be provided with one or more index algorithms 120, each index algorithm being configured to determine a general condition index that reflects the general clinical condition of the subject in a certain phase of the planned care. It is to be noted here that depending on the planned care the general clinical condition is evaluated from different points of view and therefore different physiological parameters are needed depending on the type and phase of the care.

The control and processing unit is further configured to control the display unit 121 of the apparatus. A display control algorithm may be stored in the memory of the control and processing unit and the apparatus may be provided with more than one display unit. The user may supply information and control the apparatus/system through the user interface 116, through which the runtime monitoring instances are also created. Various input information, such as patient data, may also be input through a network interface 122. Further, all the physiological parameters are not necessarily determined by the control and processing unit based on the physiological waveform signals 111 measured from the subject at the bedside, but one or more of the physiological parameters needed in the determination of the general condition index may also be received through the network interface from a laboratory, for example.

FIG. 2 is a flow diagram illustrating one embodiment of the sequence of steps to be carried out for making the apparatus ready for monitoring a subject in phased care. The monitoring apparatus is first provided with the generic monitor module (step 21), i.e. the software system from which runtime monitoring instances may be created. This may be carried out at the manufacturing stage of the apparatus, or later by providing the apparatus with a plug-in unit including the generic monitor module. Before the actual monitoring begins in a hospital, for example, a user of the apparatus creates one or more clinical applications from the generic monitor module (step 22).

For each application to be created, the user defines, for example, the type of care, the sequence of care phases related to that type of care, the parameters to be measured and monitored in each phase, and the default limits for the said parameters. For example, the type of care may be ventilator weaning, sepsis, fluid management, inotrope administration, or vasodilator administration. For ventilator weaning, for example, three different care phases may be determined: watch for readiness for spontaneous breathing, spontaneous breathing trial, and spontaneous breathing. The creation of the clinical applications from the generic monitor module may be carried out by a user having administrative rights on the apparatus. That is, an ordinary user may not normally create the clinical applications.

When the clinical applications have been created and stored in the apparatus, the actual monitoring of subjects may start. For monitoring a subject, a user, who may now be an ordinary user such as a nurse, selects a clinical application and creates a subject-specific instance from the clinical application (step 23). This may be carried out, for example, by adjusting the default limits of the parameters for the subject in question. The subject-specific instance of the clinical application is then used to monitor the subject (step 24). The subject-specific instance of the clinical application thus forms the runtime instance of the monitor module, and step 24 represents the actual on-line monitoring process. In terms of object-oriented programming, the generic monitor module may thus be regarded as a class, while a clinical application represents a sub-class and each subject-specific instance a runtime instance instantiated from a sub-class.

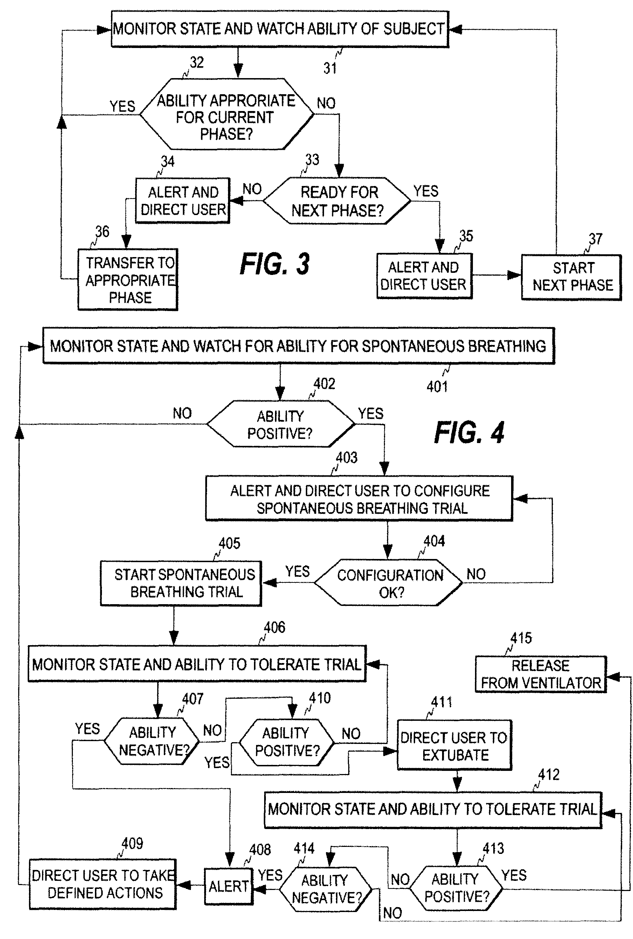

FIG. 3 illustrates one embodiment of the general structure/operation of the generic user monitor module stored in a monitoring apparatus. As discussed above, the generic monitor module is a combination of a state machine and a rule based engine in the sense that the state machine conducts the transitions between the different phases of the care and the rule based engine conducts the monitoring operation within each phase. In each phase of a clinical application, the monitoring operation carried out by the clinical application may be divided into two logical entities: the apparatus monitors the clinical state of the subject and watches the ability of the subject to receive care according to the phase (step 31). The monitoring of the clinical state involves normal monitoring; physiological parameters are derived and compared to alarm limits. The watching of the ability may involve negative and/or positive ability and may be carried out, for example, by determining at least one general condition index for the subject. Negative ability here refers to a situation in which the subject cannot be maintained in the current care phase due to a negative reason. This may be because subject is not yet able to tolerate the care given in the current phase or because the condition of the subject has deteriorated and therefore care is needed according to another care phase. Positive ability in turn refers to a situation in which it is not advisable to maintain the subject in the current care phase due to a positive reason, i.e. the subject tolerates the care of the current care phase well and is also considered to be ready for the next phase of care. In case of detected negative ability previous care phase may be restored or the subject may be transferred to a care phase that offers appropriate care in view of the changed clinical state. In some situations, when the care phase is changed, it is important to watch efficiently for signs of negative ability, since the clinical state may suffer a set-back if the subject receives care for which (s)he is not yet ready. If the subject shows no positive nor negative ability, (s)he may remain in the current care phase.

The general condition index may be determined based on a set of physiological parameters measured from the subject, and typically one index may be used to detect both negative and positive ability. The general condition index, which thus represents the ability of the subject to receive care according to the current phase, is monitored continuously. If the ability is appropriate for the current phase, i.e. the index is not too low nor too high, the subject remains in the current phase (step 32/yes). If negative ability is detected, the clinical application instance alerts and directs the user to return the subject back to the previous phase or to another phase considered appropriate for the subject (steps 33, 34 and 36). In that phase, the ability is again monitored with respect to that phase (step 31). However, the parameters and the respective parameter limits used to monitor the subject are phase-specific. As discussed above, the parameters and the respective limits are defmed in step 22 and the limits may further be adjusted in step 23. If positive ability is detected, the apparatus may inform the user of the situation and direct the user to take the steps needed for the next phase (step 35). After the user has confirmed that the care process may proceed to the next phase, the said phase is started (step 37). In the new phase, a dedicated parameter set is again used to monitor the state and ability of the subject.

The phase of the care may thus be changed according to the recommendations of the apparatus, which may depend on the general condition index of the subject, for example. Normally, user action and/or confirmation is/are needed before a new care phase can be entered. In this regard the monitor module thus resembles a state machine. Within each phase, a dedicated set of parameters, including a dedicated general condition index, are derived to monitor the clinical state of the subject and to raise alarms or give notifications to the user. In terms of a single care phase the monitor module thus resembles a rule based engine, the rules defining the alarming functionality within the phase.

At step 22, a user with administrative rights thus creates the clinical applications from the generic monitor module. For each clinical application this typically involves defining the name of the application, the sequence of the care phases, the physiological parameters to be monitored during each phase, the physiological parameters to be used for determining the general condition index during each phase, the default alarm limits and target zones of the physiological parameters for each phase, the default alarm limits of the general condition index for each phase, and the messages that are used to alert/direct the user, including the text messages related to the detected negative and positive abilities. However, the number of definitions made by the user may vary depending on the application in question.

At step 23, the default alarm limits may be adjusted to be appropriate for the subject concerned. When this is done, the runtime instance is created and the monitoring of the subject may begin according to the definitions contained in the runtime instance.

FIG. 4 illustrates an example of the monitoring operation after a clinical application has been created from the generic monitor module. The clinical application is ventilator weaning in this example. In the first phase, the ability of the subject for spontaneous breathing is watched simultaneously as the clinical state of the subject is monitored (step 401). If the general condition index indicates positive ability in this care phase (step 402/yes), the apparatus alerts and directs the user to perform the steps necessary for entering spontaneous breathing trial (step 403). This may involve reducing or disconnecting the pressure support from the ventilator. Upon user acknowledgment, the spontaneous breathing trial is started (steps 404 and 405). In response to this, the ability of the subject to tolerate the trial is monitored simultaneously as the clinical state of the subject is monitored (step 406). If the subject does not tolerate the trial (negative ability), an alarm is raised (step 408) and the apparatus directs the user to perform steps that are necessary to restore mechanical ventilation (step 409). If positive ability is detected, the user may be instructed to extubate the subject (step 411) before entering the last phase of the care, i.e. spontaneous breathing. If the subject reaches the level of positive ability, (s)he can eventually be released from the ventilator (step 415).

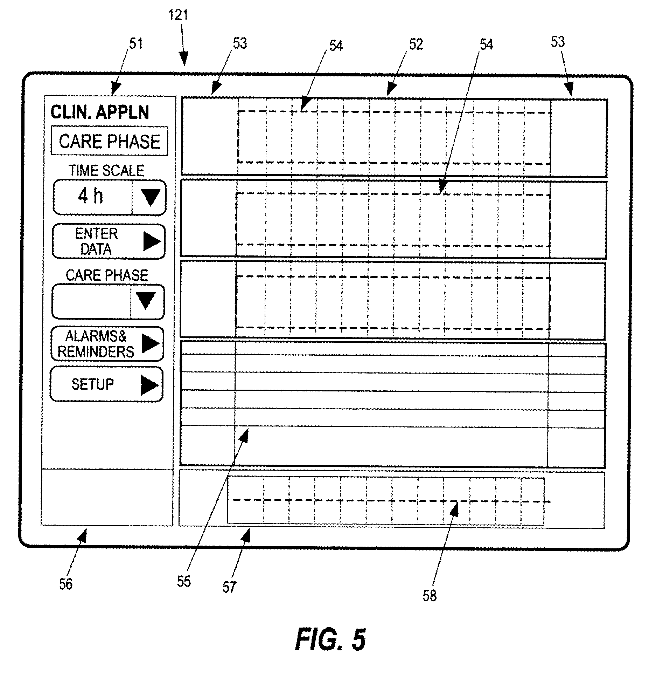

As each runtime instance may be created from the same generic module, the user interface may be made similar and visually informative for all clinical applications. FIG. 5 illustrates an example of a screen page layout of each phase of the planned care. The screen page comprises a menu field 51 and several vertically aligned trend fields 52, each comprising two scales; left scale and right scale. At the right and left ends of each trend field, a parameter field 53 indicates the particular parameter of the respective scale. In each trend field, the trends of two physiological parameters may therefore be presented. Each trend field further shows the target zones 54 of the respective parameter(s). In the figure, the target zones are shown by dashed lines, but in an actual patient monitor the area of the target zone may be coloured with a desired colour, such as green. The upper and lower limits of the target zones may be at fixed levels of the trend field. For example, the lower limit may be at a height corresponding to 10% and the upper limit at a height corresponding to 90% of the height of the trend field. The height of the target zone then corresponds to 80% of the total height of the trend field. Consequently, the height of the target zone remains fixed and the parameter values of the target zone limits are changed if the target zone is adjusted.

The menu field of FIG. 5 indicates the clinical application and the care phase. The screen page of FIG. 5 further includes an information field 55 comprising horizontal rows, each relating to a further physiological parameter whose values are shown as a series of discrete values on the respective row. For these numerical trends, the target zone may usually not be visible directly, but color coding may be used to inform the user whether the value is within or outside the target zone. All parameters displayed belong to the parameter set selected in step 22 for the clinical application in question. However, the parameters of the information field may be imported or manually entered parameters, whereas the parameters of the trend fields are more typically determined by the control and processing unit.

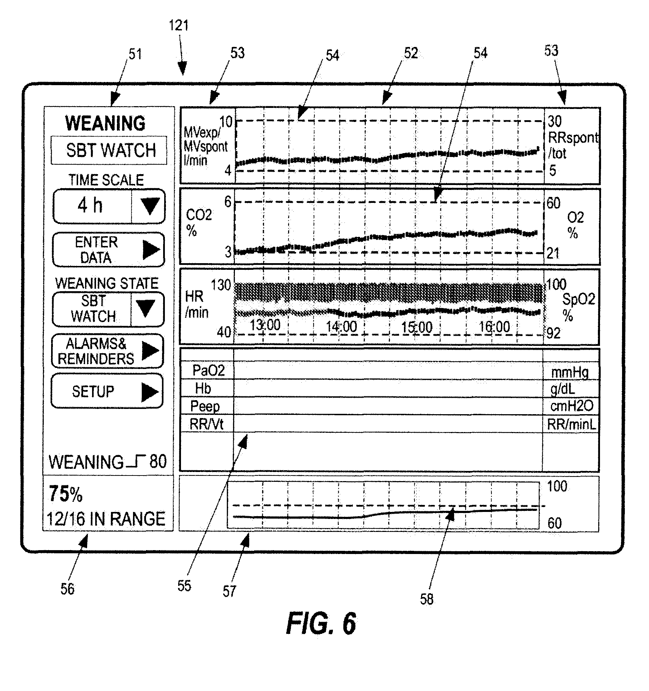

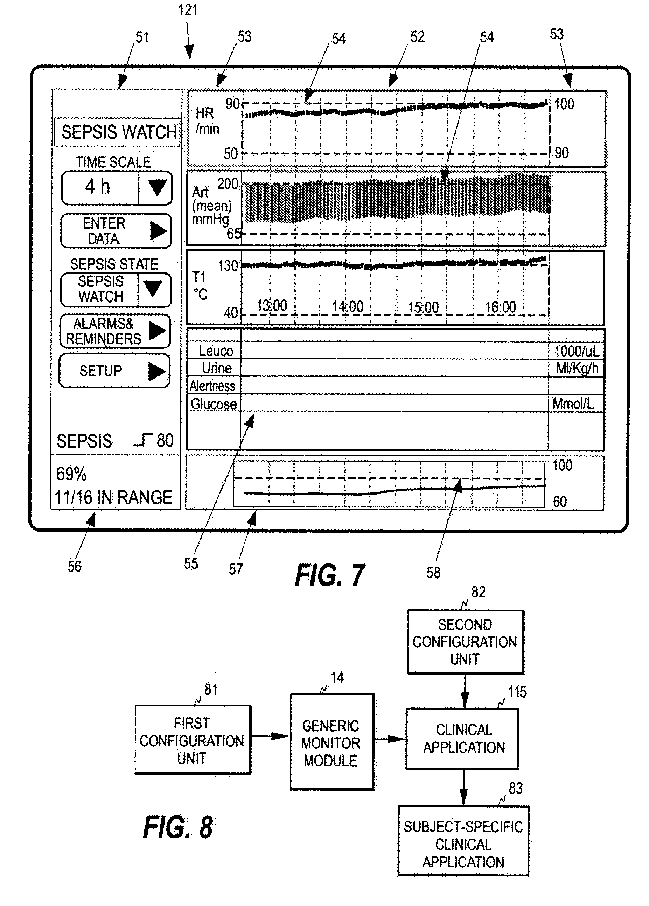

The screen page of FIG. 5 further includes an index field 56 in which the current value of the general condition index may be presented. It is assumed here that the general condition index of the subject is determined as a function of at least two integers belonging to a group including (i) the total number, M, of physiological parameters in the set of physiological parameters used for determining the general condition index, (ii) the number, L, of physiological parameters of the set that are currently within respective targeted value ranges, and (ii) the number, M-L, of physiological parameters of the set that are currently outside respective targeted value ranges. In the index field, the general condition index may be presented for example as a ratio of L/M or (M-L)/M and as a respective percentage value. The ratio may also indicate the current values of L and M, as is shown in FIGs. 6 and 7. The screen page may further include an index trend field 57 in which the trend of the general condition index may be shown, together with the respective alarm threshold 58. Each screen page thus provides quickly comprehensible information about the overall state of the subject: the trend fields indicate the clinical state and the evolution thereof with respect to vital parameters, while the index fields 56, 57 indicate the ability of the subject, and the evolution thereof, to receive care according to the phase indicated in the information field.

FIG. 6 illustrates example of a screen page displayed to the user in step 401. The menu field of FIG. 6 indicates the clinical application in question, which is ventilator weaning in this example, and the care phase, which is now spontaneous breathing trial (SBT) watch, i.e. the first phase of the respective clinical application. The lowermost trend field, for example, shows the trends of two physiological parameters: Sp02 on the right scale and heart rate (HR) on the left scale. The Sp02 value is shown as a bar extending downwards from the value of 100. The threshold value of the general condition index is 80% in this example, and the trend indicates that the general clinical condition of the subject is not yet good enough for a spontaneous breathing trial (12 out of 16 parameters of the general condition index are within respective target zones). If the index exceeds the threshold value, the apparatus alerts and directs the user to perform the steps necessary for entering the next care phase, cf. step 403 of FIG. 4.

FIG. 7 illustrates another example of a screen page displayed to the user in step 401. The menu field of FIG. 7 indicates the clinical application in question, which is sepsis in this example, and the care phase, which is now sepsis watch, i.e. the first phase of the clinical application intended for caring sepsis. In case of sepsis, three phases may be used, for example: watch for sepsis, watch for severe sepsis, and severe sepsis. As can be seen from the examples, the parameters differ, but the clinical state and the general condition in view of the care phase is presented in similar way regardless of the application. For the fluid management application mentioned above, at least three care phases may be used: watch for hypovolemia, fluid challenge, and at least one monitor phase subsequent to the fluid challenge phase. If hypovolemia is detected in the first phase, the underlying reasons may be examined in the fluid challenge phase by giving the subject a fluid bolus, for example. The selected monitor phase may depend on the outcome of the fluid challenge phase.

In terms of the monitoring of the subject, the control and processing unit 112 of FIG. 1 may be seen as an entity of three modules or units that are needed to obtain a runtime instance of a clinical application. With reference to FIG. 8, the control and processing unit includes the generic monitor module 14 serving as a template for creating runtime instances. A user may create a clinical application 115 by configuring the generic monitor module by a first configuration unit 81. The result obtained is a clinical application 115. A second configuration unit 82 allows the user to create a subject-specific clinical application 83 from the clinical application. As discussed above, the generic monitor module may be seen as a monitor class from which sub-classes 115 and further runtime instances 83 may be constructed.

A conventional patient monitor may also be upgraded to enable the users of the monitor to create subject-specific clinical applications. Such an upgrade may be implemented, for example, by delivering to the monitor a plug-in unit that may include the software system with the generic monitor module. The plug-in unit may be delivered, for example, on a data carrier, such as a CD or a memory card, or the through a telecommunications network. Regardless of whether or not the software of the control and processing unit is upgradable, the control and processing unit may also utilize physiological parameters transferred from an external entity, such as a laboratory or an external data system. The set of physiological parameters selected for the type of care to be applied to the subject may thus include internally determined parameters and/or parameters imported from an external entity. The apparatus may also be implemented as an auxiliary apparatus/unit connectable to an existing patient monitor. In this embodiment, the auxiliary apparatus/unit may be used to create the clinical applications from the generic module serving as a template.

This written description uses examples to disclose the invention, including the best mode, and also to enable any p15erson skilled in the art to make and use the invention. The patentable scope of the invention is defmed by the claims, and may include other examples that occur to those skilled in the art. Such other examples are intended to be within the scope of the claims if they have structural or operational elements that do not differ from the literal language of the claims, or if they have structural or operational elements with insubstantial differences from the literal language of the claims.
Various aspects and embodiments of the present invention are defmed by the following numbered clauses:
1. A method for monitoring a subject receiving care, the method comprising:
   - providing a monitoring apparatus with a generic monitor module serving as a template for creation of monitoring instances;
   - creating at least one clinical application from the generic monitor module, wherein each clinical application defines a care process for caring a subject;
   - producing a subject-specific monitoring instance from a clinical application, thereby to obtain a runtime monitoring instance, wherein the clinical application is any of the at least one clinical application; and
   - employing the runtime monitoring instance for monitoring the subject online.
2. The method according to clause 1, wherein the creating includes defming a sequence of care phases for each of the at least one clinical application.
3. The method according to clause 1 or clause 2, wherein the creating further includes defining a first set of physiological parameters to be monitored in each of the care phases.
4. The method according to any preceding clause, wherein the creating further includes defining a general condition index representing ability of the subject to receive care, wherein the defining is performed for at least one of the care phases.
5. The method according to any preceding clause, wherein the defming further includes
   - defining a second set of physiological parameters;
   - defining a targeted value range for each physiological parameter in the second set of physiological parameters; and
   - defining the general condition index as a function of at least two integers belonging to a group including (i) number of physiological parameters of the second set that are currently within respective targeted value ranges, (ii) number of physiological parameters of the second set that are currently outside respective targeted value ranges, and (iii) total number of physiological parameters in the second set of physiological parameters
6. The method according to any preceding clause, wherein the creating further includes defining default alarm limits for the first set of physiological parameters.
7. The method according to any preceding clause, wherein the producing includes adjusting at least part of the default limits for the subject.
8. An apparatus for monitoring a subject receiving care, the apparatus comprising:
   - a generic monitor module serving as a template for creation of monitoring instances;
   - a first configuration module adapted to allow a user to create at least one clinical application from the generic monitor module, wherein each clinical application defines a care process for caring a subject;
   - a second configuration module adapted to allow a user to create a subject-specific monitoring instance from a clinical application, thereby to obtain a runtime monitoring instance, wherein the clinical application is any of the at least one clinical application,
      wherein the apparatus is configured to execute the runtime monitoring instance for monitoring the subject online.
9. The apparatus according to clause 8, wherein the first configuration module is adapted to allow the user to create a sequence of care phases for each of the at least one clinical application.
10. The apparatus according to clause 8 or clause 9, wherein the first configuration module is further adapted to allow the user to defme a first set of physiological parameters to be monitored in each of the care phases.
11. The apparatus according to any of clauses 8 to 10, wherein the first configuration module is further adapted to allow the user to defme, for at least one of the care phases, a general condition index representing ability of the subject to receive care.
12. The apparatus according to any of clauses 8 to 11, wherein the first configuration module is adapted to allow the user to
   - define a second set of physiological parameters;
   - define a targeted value range for each physiological parameter in the second set of physiological parameters; and
   - define the general condition index as a function of at least two integers belonging to a group including (i) number of physiological parameters of the second set that are currently within respective targeted value ranges, (ii) number of physiological parameters of the second set that are currently outside respective targeted value ranges, and (iii) total number of physiological parameters in the second set of physiological parameters.
13. The apparatus according to any of clauses 8 to 12, wherein the first configuration module is adapted to allow the user to define default alarm limits for the first set of physiological parameters.
14. The apparatus according to any of clauses 8 to 13, wherein the second configuration module is adapted to allow the user to adjust at least part of the default limits for the subject.
15. A computer program product for monitoring a subject receiving care, the computer program product comprising:
   - a generic monitor module serving as a template for creation of monitoring instances;
   - a first program product portion adapted to allow a user to create at least one clinical application from the generic monitor module, wherein each clinical application defines a care process for caring a subject; and
   - a second program product portion adapted to allow a user to create a subject-specific monitoring instance from the clinical application, thereby to obtain a runtime monitoring instance, wherein the clinical application is any of the at least one clinical application and the runtime monitoring instance is executable by a monitoring apparatus for monitoring the subject online.
16. The computer program product according to clause 15, wherein the first program product portion is adapted to allow the user to create a sequence of care phases for each of the at least one clinical application.
17. The computer program product according to clause 15 or clause 16, wherein the first program product portion is further adapted to allow the user to define a first set of physiological parameters to be monitored in each of the care phases.
18. The computer program product according to any of clauses 15 to 17, wherein the first program product portion is further adapted to allow the user to define, for at least one of the care phases, a general condition index representing ability of the subject to receive care.
19. The computer program product according to any of clauses 15 to 18, wherein the first program product portion is adapted to allow the user to
   - defme a second set of physiological parameters;
   - define a targeted value range for each physiological parameter in the second set of physiological parameters; and
   - defme the general condition index as a function of at least two integers belonging to a group including (i) number of physiological parameters of the second set that are currently within respective targeted value ranges, (ii) number of physiological parameters of the second set that are currently outside respective targeted value ranges, and (iii) total number of physiological parameters in the second set of physiological parameters.
20. The computer program product according to any of clauses 15 to 19, wherein the first program product portion is adapted to allow the user to defme default alarm limits for the first set of physiological parameters.

## Claims

1. A method for monitoring a subject receiving care, the method comprising:
- providing a monitoring apparatus (100) with a generic monitor module (114) serving as a template for creation of monitoring instances;
- creating at least one clinical application (115) from the generic monitor module, wherein each clinical application defines a care process for caring a subject (110);
- producing a subject-specific monitoring instance (83) from a clinical application, thereby to obtain a runtime monitoring instance, wherein the clinical application is any of the at least one clinical application; and
- employing the runtime monitoring instance (83) for monitoring the subject (110) online.

2. The method according to claim 1, wherein the creating includes defining a sequence of care phases for each of the at least one clinical application (115).

3. The method according to claim 1 or claim 2, wherein the creating further includes defining a first set of physiological parameters to be monitored in each of the care phases.

4. The method according to any preceding claim, wherein the creating further includes defining a general condition index representing ability of the subject to receive care, wherein the defining is performed for at least one of the care phases.

5. The method according to any preceding claim, wherein
- the creating further includes defining default alarm limits for the first set of physiological parameters; and
- the producing includes adjusting at least part of the default limits for the subject (110).

6. An apparatus (100) for monitoring a subject receiving care, the apparatus comprising:
- a generic monitor module (114) serving as a template for creation of monitoring instances;
- a first configuration module adapted to allow a user to create at least one clinical application (115) from the generic monitor module, wherein each clinical application defines a care process for caring a subject;
- a second configuration module adapted to allow a user to create a subject-specific monitoring instance (83) from a clinical application, thereby to obtain a runtime monitoring instance, wherein the clinical application is any of the at least one clinical application,
wherein the apparatus is configured to execute the runtime monitoring instance (83) for monitoring the subject (110) online.

7. The apparatus according to claim 6, wherein the first configuration module is adapted to allow the user to create a sequence of care phases for each of the at least one clinical application (115).

8. The apparatus according to claim 6 or claim 7, wherein the first configuration module is further adapted to allow the user to define a first set of physiological parameters to be monitored in each of the care phases.

9. The apparatus according to any of claims 6 to 8, wherein the first configuration module is further adapted to allow the user to define, for at least one of the care phases, a general condition index representing ability of the subject to receive care.

10. The apparatus according to any of claims 6 to 9, wherein the first configuration module is adapted to allow the user to
- define a second set of physiological parameters;
- define a targeted value range for each physiological parameter in the second set of physiological parameters; and
- defme the general condition index as a function of at least two integers belonging to a group including (i) number of physiological parameters of the second set that are currently within respective targeted value ranges, (ii) number of physiological parameters of the second set that are currently outside respective targeted value ranges, and (iii) total number of physiological parameters in the second set of physiological parameters.

11. The apparatus according to any of claims 6 to 10, wherein
- the first configuration module is adapted to allow the user to define default alarm limits for the first set of physiological parameters; and
- the second configuration module is adapted to allow the user to adjust at least part of the default limits for the subject (110).

12. A computer program product for monitoring a subject receiving care, the computer program product comprising:
- a generic monitor module (114) serving as a template for creation of monitoring instances;
- a first program product portion adapted to allow a user to create at least one clinical application (115) from the generic monitor module (114), wherein each clinical application defines a care process for caring a subject (110); and
- a second program product portion adapted to allow a user to create a subject-specific monitoring instance (83) from the clinical application, thereby to obtain a runtime monitoring instance, wherein the clinical application is any of the at least one clinical application and the runtime monitoring instance (83) is executable by a monitoring apparatus for monitoring the subject (110) online.

13. The computer program product according to claim 12, wherein the first program product portion is adapted to allow the user to create a sequence of care phases for each of the at least one clinical application (115).

14. The computer program product according to claim 12 or claim 13, wherein the first program product portion is further adapted to allow the user to define a first set of physiological parameters to be monitored in each of the care phases.

15. The computer program product according to any of claims 12 to 14, wherein the first program product portion is further adapted to allow the user to define, for at least one of the care phases, a general condition index representing ability of the subject to receive care.
